**Europäisches Patentamt**

**European Patent Office**

**Office européen des brevets**

(11) Veröffentlichungsnummer: **0 381 048**

**A1**

(12) # EUROPÄISCHE PATENTANMELDUNG

(21) Anmeldenummer: **90101466.2**

(22) Anmeldetag: **25.01.90**

(51) Int. Cl.5: **C07C 315/02, C07C 317/14**

(30) Priorität: **01.02.89 DE 3902896**

(43) Veröffentlichungstag der Anmeldung:
**08.08.90 Patentblatt 90/32**

(84) Benannte Vertragsstaaten:
**DE FR GB**

(71) Anmelder: **BASF Aktiengesellschaft**
**Carl-Bosch-Strasse 38**
**D-6700 Ludwigshafen(DE)**

(72) Erfinder: **Stumpp, Michael, Dr.**
**An der Marlach 13**
**D-6705 Deidesheim(DE)**
Erfinder: **Neumann, Peter, Dr.**
**Poststrasse 28**
**D-6800 Mannheim 31(DE)**
Erfinder: **Schaefer, Gerhard, Dr.**
**Schafrippel 2**
**D-6900 Heidelberg(DE)**

(54) **Verfahren zur Herstellung von Bis-(4-bromphenyl)-sulfon.**

(57) Verfahren zur Herstellung von Bis-(4-bromphe-nyl)-sulfon, indem man Diphenylsulfoxid in Anwesenheit von Eisessig und Wasser bei 80 bis 100°C bromiert und die erhaltene Reaktionslösung durch Zugabe eines Oxidationsmittels umsetzt.

EP 0 381 048 A1

## Verfahren zur Herstellung von Bis-(4-bromphenyl)-sulfon

Diese Erfindung betrifft ein neues und verbessertes Verfahren zur Herstellung von Bis-(4-bromphenyl)-sulfon durch Bromierung von Diphenylsulfoxid und anschließende Oxidation.

Bis-(4-bromphenyl)-sulfon ist ein wichtiges Zwischenprodukt, das zur Herstellung aromatischer Polysulfone und zur Synthese von Bis-(4-aminophenyl)-sulfon, das man sowohl für die Lepratherapie als auch zur Härtung von Epoxidharzen benötigt, verwendet wird.

Aus Bull. Chem. Soc. Jpn., 39, 614-17 (1966) ist bekannt, daß bei der Umsetzung von Diphenylsulfoxid mit Brom in Anwesenheit von Eisessig und Wasser Bis(4-bromphenyl)-sulfid gebildet wird.

Man kann Bis-(4-bromphenyl)-sulfon z.B. nach Synthesis, 561-562 (1972) durch Oxidation von Bis-(4-bromphenyl)-sulfid mit Cerammoniumnitrat und anschließende Oxidation des auf diese Weise erhaltenen Bis-(4-bromphenyl)-sulfoxids mit stärkeren Oxidationsmitteln, wie Kaliumbromat (Indian J. Chem., Sect. A 1986, 25A (7), 678-80) oder Peroxomonosulfat (Indian Acad. Sci., Chem. Sci., 1986, 97 (5-6), 555-63) herstellen.

Der Erfindung lag die Aufgabe zugrunde, ein neues und verbessertes Verfahren zur Herstellung von Bis-(4-bromphenyl)-sulfon zu entwickeln.

Demgemäß wurde ein neues und verbessertes Verfahren zur Herstellung von Bis-(4-bromphenyl)-sulfon gefunden, welches dadurch gekennzeichnet ist, daß man Diphenylsulfoxid in Anwesenheit von Eisessig und Wasser bei 80 bis 100 °C bromiert und die erhaltene Reaktionslösung durch Zugabe eines Oxidationsmittels umsetzt.

Die erhaltene Reaktionslösung enthält hauptsächlich Wasser, Eisessig, Bromid und Bis-(4bromphenyl)-sulfid und kann ohne Isolierung einer Zwischenverbindung durch Zugabe eines Oxidationsmittels oxidiert werden, wobei das entstandene Sulfid zum Bis-(4-bromphenyl)-sulfon und das Bromid zu Brom oxidiert wird und zurückgewonnen werden kann.

Das erfindungsgemäße Verfahren kann wie folgt durchgeführt werden:

Das Diphenylsulfoxid kann zusammen mit Eisessig und Wasser in ein Reaktionsgefäß vorgelegt werden. Die Menge des Lösungsmittels beträgt zwischen der ein- und 50-fachen, in der Regel etwa der zehnfachen Gewichtsmenge des eingesetzten Diphenylsulfoxids. Das Verhältnis Eisessig zu Wasser beträgt zwischen 1:10 und 100:1, vorzugsweise 1:4 bis 30:1, besonders bevorzugt 5:1 bis 20:1, in der Regel 10:1. Diese Parameter sind jedoch nicht Voraussetzung für eine optimale Durchführung der Synthese. Brom kann entweder vor, nach oder während des Aufheizens des Reaktionsgemisches auf 80 bis 100 °C zugegeben werden. Die Menge des eingesetzten Broms beträgt 0,7 bis 5 Moläquivalent, vorzugsweise 0,9 bis 2, in der Regel äquimolar zum eingesetzten Diphenylsulfoxid. Nach Erreichen der Reaktionstemperatur hält man die Temperatur ca 1 bis 6 Stunden, vorteilhafterweise 3 Stunden bei 80 bis 100 °C. Zur Oxidation werden anschließend starke Oxidationsmittel, wie organische oder anorganische Persäuren wie z.B. m-Chlorperbenzoesäure oder Wasserstoffperoxid eingesetzt. Vorteilhafterweise empfiehlt sich die Verwendung von Wasserstoffperoxid, das im Überschuß von 2 bis 10 Moläquivalent, vorzugsweise 3 bis 6 Moläquivalent des eingesetzten Diphenylsulfoxids verwendet wird. Das Oxidationsmittel wird portionsweise zugesetzt, ohne die Reaktionstemperatur zu erniedrigen. Das entstehende Brom wird abdestilliert und kondensiert. Nach einer Reaktionszeit von 1 bis 3 Stunden wird mit Wasser verdünnt, abgekühlt, abfiltriert und das Rohprodukt mehrmals mit Wasser gewaschen. Das auf diese Weise erhaltene Rohprodukt kann nach üblichen Verfahren, wie z.B. Umkristallisation aus Alkoholen gereinigt werden.

Beispiel

Zu einem Gemisch aus 28 g (0,138 Mol) Diphenylsulfoxid, 20 ml Wasser und 300 ml Eisessig wurden innerhalb 15 Minuten 22 g (0,138 Mol) Brom zugetropft. Anschließend wurde die Innentemperatur auf 90 °C erhöht und 3 Stunden bei dieser Temperatur gerührt. Danach wurden innerhalb 30 Minuten 50 ml einer 30 %igen Wasserstoffperoxidlösung zugetropft. Das bei der Reaktion frei werdende Brom wurde aus der Lösung abdestilliert und kondensiert. Nach 2 Stunden Reaktionszeit wurde mit 500 ml Wasser verdünnt und das Rohprodukt bei Raumtemperatur abgesaugt, 2 mal mit 500 ml Wasser gewaschen und getrocknet. Man erhielt 25,1 g (0,115 Mol; 83 %) Bis-(4-bromphenyl)-sulfon in einer Reinheit von 98 % (GC).

**Ansprüche**

1. Verfahren zur Herstellung von Bis-(4-bromphenyl)-sulfon, dadurch gekennzeichnet, daß man Diphenylsulfoxid in Anwesenheit von Eisessig und Wasser bei 80 bis 100 °C bromiert und die erhaltene Reaktionslösung durch Zugabe eines Oxidationsmittels umsetzt.

2. Verfahren nach Anspruch 1, dadurch gekennzeichnet, daß man als Oxidationsmittel ein Peroxid verwendet.

3. Verfahren nach Anspruch 1, dadurch gekennzeichnet, daß man Wasserstoffperoxid als Oxidationsmittel verwendet.

Europäisches Patentamt

EUROPÄISCHER RECHERCHENBERICHT

Nummer der Anmeldung

EP 90 10 1466

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | CHEMICAL ABSTRACTS, Band 111, Nr. 19, 6. November 1989, Seite 689, Zusammenfassung Nr. 173756q, Columbus, Ohio, US; & JP-A-01 106 858 (TEC. CHEM. K.K.) 24-04-1989 --- | | C 07 C 315/02 C 07 C 317/14 |
| D,A | BULLETIN OF THE CHEMICAL SOCIETY OF JAPAN, Band 39, Nr. 3, 1966, Seiten 614-617, Tokyo, JP; W. TAGAKI et al.: "The reaction of diphenyl sulfoxide with bromine in aqueous acetic acid" --- | | |
| D,A | PROCEEDINGS OF THE INDIAN ACADEMY OF SCIENCES, CHEMICAL SCIENCES, Band 97, Nr. 5,6, Dezember 1986, Seiten 555-563, IN; R. SUTHAKARAN et al.: "Mechanism of the oxidation of alkyl aryl and dipenyl sulphoxides by peroxomonosulphate" --- | | |
| D,A | INDIAN JOURNAL OF CHEMISTRY, Band 25A, Juli 1986, Seiten 678-680, IN; C. SRINIVASAN et al.: "Mechanism of oxidation of aryl methyl sulphoxides & diaryl sulphoxides by potassium bromate" ----- | | RECHERCHIERTE SACHGEBIETE (Int. Cl.5) <br><br> C 07 C 315/00 <br> C 07 C 317/00 |

Der vorliegende Recherchenbericht wurde für alle Patentansprüche erstellt

| Recherchenort | Abschlußdatum der Recherche | Prüfer |
|---|---|---|
| DEN HAAG | 20-04-1990 | ZAROKOSTAS K. |